# EUROPÄISCHE PATENTANMELDUNG

(11) **EP 3 835 592 A1**
(43) Veröffentlichungstag der Anmeldung: **16.06.2021**
(21) Anmeldenummer: 20020596.1
(22) Anmeldetag: 08.12.2020
(51) Int. Cl.: F04D 29/28, F04D 29/30

(54) **LÜFTERRAD MIT REDUZIERTER MASSENTRÄGHEIT FÜR EIN ATEMTHERAPIEGERÄT**

(30) Priorität: 09.12.2019 DE 102019008521
(71) Anmelder: Löwenstein Medical Technology S.A., 2557 Luxembourg (LU)
(72) Erfinder: Gerlach, Angela, 22549 Hamburg (DE)
(74) Vertreter: Marx, Thomas

(57) **Zusammenfassung**

Die vorliegende Erfindung betrifft ein Atemtherapiegerät mit einer Lüftereinrichtung zum Erzeugen einer Atemluftströmung für die Durchführung einer Atemtherapie. Die Lüftereinrichtung umfasst wenigstens ein drehbares Lüfterrad mit einer Mehrzahl von Schaufelelementen die auf einer Tragscheibe angeordnet sind. Dabei weisen die Schaufelelemente und/oder die Tragscheibe wenigstens eine Aussparung auf.

## Beschreibung

Die vorliegende Erfindung betrifft ein Atemtherapiegerät mit wenigstens einer Lüftereinrichtung zum Erzeugen einer Atemluftströmung für die Durchführung einer Atemtherapie. Die Lüftereinrichtung umfasst wenigstens ein drehbares Lüfterrad mit einer Mehrzahl von Schaufelelementen.

Solche Geräte werden beispielsweise zur Beatmung bzw. Atmungsunterstützung oder zur Hustenunterstützung eingesetzt. Damit die Behandlung bzw. Therapie nicht als störend empfunden wird, sollten die Betriebsgeräusche des Lüfters möglichst gering sein. Für eine gezielte Atemtherapie ist es zudem entscheidend, dass möglichst zügige und kurzfristige Drehzahlanpassungen des Lüfterrades erfolgen können.

Demgegenüber ist es die Aufgabe der vorliegenden Erfindung, ein Atemtherapiegerät zur Verfügung zu stellen, welches die zuvor genannten Anforderungen möglichst vorteilhaft erfüllen kann.

Diese Aufgabe wird mit einem Atemtherapiegerät des Anspruchs 1 sowie mit einem Lüfterrad gemäß Anspruch 26 gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Das erfindungsgemäße Atemtherapiegerät umfasst wenigstens eine Lüftereinrichtung zum Erzeugen einer Atemluftströmung. Die Lüftereinrichtung umfasst wenigstens ein drehbares Lüfterrad. Das Lüfterrad umfasst eine Mehrzahl von Schaufelelementen auf einer Tragscheibe und zumindest eine Aussparung.

Die Aufgabe wird insbesondere gelöst durch ein
Atemtherapiegerät mit wenigstens einer Lüftereinrichtung zum Erzeugen einer Atemluftströmung für die Durchführung einer Atemtherapie, wobei die Lüftereinrichtung wenigstens ein drehbares Lüfterrad mit einer Nabe aufweist, die sich im Mittelpunkt des Lüfterrades befindet, mit einer Tragscheibe, die sich ausgehend von der Nabe in radialer Richtung erstreckt und wobei die Tragscheibe einen Außenradius aufweist, der einen Umfang definiert, mit einer Mehrzahl von Schaufelelementen die mit ihrer Basis auf der Tragscheibe angeordnet sind und sich ausgehend von der Nabe in radialer Richtung zu ihrer Schaufelspitze erstrecken und sich ausgehend von der Basis an der Deckscheibe in axialer Richtung erstrecken und dort eine Längsseite definieren, wobei die Schaufelelementen den Schaufelspitzen zumindest einen Außenradius aufweisen der den Außenumfang der Schaufelelemente definiert dadurch gekennzeichnet, dass die Schaufelelemente und/oder die Tragscheibe wenigstens eine Aussparung aufweisen.

Die Aufgabe wird auch dadurch gelöst, dass die Aussparung in dem Material des Lüfterrades ausgebildet ist.

Die Aufgabe wird auch dadurch gelöst, dass mehrere Aussparungen in Umfangsrichtung des Lüfterrades nebeneinander ausgebildet sind.

Die Aufgabe wird auch dadurch gelöst, dass mehrere Aussparungen in Radialrichtung des Lüfterrades hintereinander ausgebildet sind.

Die Aufgabe wird auch dadurch gelöst, dass die Aussparung in der Tragscheibe ausgebildet ist. Die Aufgabe wird auch dadurch gelöst, dass zumindest ein Durchgansloch in der Tragscheibe zwischen zwei Schaufelelementen ausgebildet ist.

Die Aufgabe wird auch dadurch gelöst, dass die Schaufelelemente innerhalb eines Umfangs der Scheibe angeordnet sind und/oder wobei die Schaufelelemente nicht über den Umfang der Scheibe hinausstehen.

Die Aufgabe wird auch dadurch gelöst, dass die Aussparung in dem Material des Schaufelelementes ausgebildet ist.

Die Aufgabe wird auch dadurch gelöst, dass die Aussparung an der Schaufelspitze ausgebildet ist und daher der Außenradius geringer ist als der Außenradius der Tragscheibe.

Die Aufgabe wird auch dadurch gelöst, dass der Umfang der Tragscheibe größer ist als der Umfang der Schaufelelemente.

Die Aufgabe wird auch dadurch gelöst, dass die Aussparung in dem Außenradius der Tragscheibe ausgebildet ist und daher der Außenradius geringer ist als der Außenradius der Schaufelelemente.

Die Aufgabe wird auch dadurch gelöst, dass der Umfang der Schaufelelemente größer ist als der Umfang der Tragscheibe.

Die Aufgabe wird auch dadurch gelöst, dass die Aussparung an der Schaufelspitze derart ausgebildet und angeordnet ist, dass die Schaufelspitze in axialer Richtung abgeschrägt ist.

Die Aufgabe wird auch dadurch gelöst, dass die axiale Abschrägung in einem Bereich zwischen 1° und 50° liegt.

Die Aufgabe wird auch dadurch gelöst, dass die axiale Abschrägung in einem Bereich zwischen 1° und 25° liegt.

Die Aufgabe wird auch dadurch gelöst, dass die axiale Abschrägung in einem Bereich zwischen 2° und 20° liegt.

Die Aufgabe wird auch dadurch gelöst, dass die axiale Abschrägung in einem Bereich zwischen 3° und 16° liegt.

Die Aufgabe wird auch dadurch gelöst, dass die axiale Abschrägung in einem Bereich zwischen 4° und 7° liegt.

Die Aufgabe wird auch dadurch gelöst, dass die Aussparung an der Schaufelspitze derart ausgebildet und angeordnet ist, dass die Schaufelspitze in axialer Richtung gekrümmt oder gebogen ist.

Die Aufgabe wird auch dadurch gelöst, dass die Aussparung an der Schaufelspitze derart ausgebildet und angeordnet ist, dass das Schaufelelement an der Schaufelspitze zumindest zwei unterschiedliche Außenradien aufweist.

Die Aufgabe wird auch dadurch gelöst, dass ein geringerer Außenradius des Schaufelelementes an der Schaufelspitze an der oder nahe der Basis des Schaufelelementes angeordnet ist.

Die Aufgabe wird auch dadurch gelöst, dass ein geringerer Außenradius des Schaufelelementes an der Schaufelspitze an der oder nahe der Längsseite des Schaufelelementes angeordnet ist.

Die Aufgabe wird auch dadurch gelöst, dass ein geringerer Außenradius zwischen der Längsseite und der Basis des Schaufelelementes angeordnet ist.

Die Aufgabe wird auch dadurch gelöst, dass das Lüfterrad einstückig durch ein Spritzgussverfahren hergestellt ist.

Die Aufgabe wird auch dadurch gelöst, dass die Aussparungen eine geringere Materialstärke aufweisen als die Scheibe oder die Schaufelelemente. Die Aussparungen können auch als Durchgangslöcher ausgebildet sein.

Die Aufgabe wird auch gelöst durch ein Lüfterrad für ein Atemtherapiegerät, umfassend eine Mehrzahl von Schaufelelementen die auf einer Tragscheibe angeordnet sind, dadurch gekennzeichnet, dass die Schaufelelemente und/oder die Tragscheibe wenigstens eine Aussparung aufweisen.

Das erfindungsgemäße Atemtherapiegerät bietet viele Vorteile.

So ist das Lüfterrad der Erfindung erheblich leichter als ein Lüfterrad nach dem SdT. Dadurch weist das Lüfterrad eine besonders geringe Trägheit auf, sodass Drehzahlanpassungen besonders zügig und mit einem geringen Leistungsaufwand möglich sind.

Zudem kann die Lüftereinrichtung der Erfindung im Vergleich zu herkömmlichen Lüftern für Atemtherapiegeräte bei gleichem Druck mit geringeren Drehzahlen betrieben werden. Das bringt wiederum erheblich geringere Betriebsgeräusche mit sich, sodass eine besonders leise Therapie erfolgen kann. Zudem hat sich herausgestellt, dass mit der Erfindung auch ein erheblich verbesserter Wirkungsgrad für die Erzeugung der Atemluftströmung erreicht wird. Die Erfindung bietet daher erhebliche Vorteile gegenüber anderen Ansätzen.

In einer vorteilhaften Ausgestaltung kann das Lüfterrad an wenigstens einer axialen Seite, vorzugsweise an nur einer axialen Seite, mit wenigstens einer Scheibe ausgestattet sein. Vorzugsweise ist die Scheibe als Tragscheibe zur wenigstens teilweisen Befestigung der Schaufelelemente geeignet und ausgebildet. Möglich ist auch, dass die Scheibe als Deckscheibe zur wenigstens teilweisen strömungstechnischen Abdeckung der Schaufelelemente geeignet und ausgebildet ist.

Die Scheibe kann fest mit dem Lüfterrad verbunden sein. Möglich ist auch, dass die Scheibe separat zum Lüfterrad ausgeführt ist bzw. ein vom Lüfterrad getrenntes Bauteil darstellt. Insbesondere ist die Tragscheibe fest mit dem Lüfterrad verbunden und/oder die Deckscheibe separat zum Lüfterrad ausgebildet.

Die Scheibe, insbesondere die Deckscheibe und/oder die Tragscheibe, weist insbesondere eine radial innen liegende Ansaugöffnung und/oder Ausströmöffnung auf. Es ist möglich, dass die Schaufelelemente über den Umfang der Deckscheibe hinausragen. Die Deckscheibe ist insbesondere nicht mit den Schaufelelementen verbunden. Die Deckscheibe dient insbesondere nicht zur Befestigung der Schaufelelemente.

Die Scheibe, insbesondere die Tragscheibe und/oder die Deckscheibe, trägt die Schaufelelemente. Die Tragscheibe ist insbesondere fest mit den Schaufelelementen verbunden und vorzugsweise einstückig mit den Schaufelelementen ausgebildet. Insbesondere weist die Tragscheibe eine zentrale Ausnehmung auf, beispielsweise als Teil einer Nabe zum Durchführen einer Antriebswelle. Die Schaufelelemente können über den Umfang der Tragscheibe hinausragen. Die Scheibe kann geschlossen ausgebildet sein.

Die Schaufelelemente sind vorzugsweise innerhalb eines Umfangs der Scheibe angeordnet. Insbesondere stehen die Schaufelelemente nicht über den Umfang der Scheibe hinaus. Möglich ist aber auch, dass die Schaufelelemente über den Umfang der Scheibe hinausragen. In einer besonders vorteilhaften Ausgestaltung sind die Schaufelelemente wenigstens teilweise und vorzugsweise vollständig gerade ausgebildet. Das ermöglicht eine besonders unaufwendige und wirtschaftliche Herstellung des Lüfterrades, beispielsweise mittels eines Spritzgussverfahrens oder dergleichen. Insbesondere sind die Schaufelelemente eben bzw. planar ausgebildet. Insbesondere stehen die Flächen der Schaufelelemente rechtwinkelig auf der Hauptebene der Tragscheibe. Beispielsweise sind sogenannte 90°-Schaufeln vorgesehen. Es können auch 90°-Schaufeln +/- 15° vorgesehen sein. Möglich und bevorzugt ist auch, dass die Schaufelelemente gekrümmt ausgebildet sind. Die Schaufelelemente können über ihre Längsausdehnung verdreht bzw. helixartig ausgebildet sein. Die Schaufelelemente können auch gebogen sein, ohne dabei um ihre Längsachse verdreht zu sein.

Insbesondere sind die Schaufelelemente des Lüfterrades alle identisch ausgebildet. Möglich ist aber auch, dass die Schaufelelemente des Lüfterrades unterschiedlich ausgebildet sind. Insbesondere sind die Schaufelelemente dabei wahlweise, beispielsweise abwechselnd, mit den zuvor genannten Merkmalen ausgebildet.

Vorzugsweise ist das Lüfterrad durch wenigstens ein Spritzgussverfahren hergestellt bzw. herstellbar. Es können auch andere geeignete Verfahren zur Herstellung vorgesehen sein. Insbesondere sind die Schaufelelemente und/oder die Scheibe und/oder die Nabe derartig hergestellt. Das Lüfterrad kann aus Kunststoff oder Metall oder auch einem Verbundwerkstoff gefertigt sein.

Das Lüfterrad ist insbesondere einstückig ausgeführt. Insbesondere sind die Schaufelelemente und die Scheibe und insbesondere auch die Nabe einstückig miteinander hergestellt und verbunden. Möglich ist auch, dass das Lüfterrad einstückig mit der Tragscheibe und/oder Deckscheibe verbunden ist. Das Lüfterrad kann auch mehrteilig ausgeführt sein.

Das erfindungsgemäße Lüfterrad ist für ein Atemtherapiegerät vorgesehen, wie es vorzugsweise zuvor beschrieben wurde. Das Lüfterrad umfasst eine Mehrzahl von Schaufelelementen. Dabei ist das Lüfterrad vorzugsweise wie zuvor für das erfindungsgemäße Atemtherapiegerät beschrieben ausgebildet.

Die hier vorgestellte Erfindung kann insbesondere bei allen geeigneten Formen von Lüfterrädern angewendet werden. Das Lüfterrad kann als Sternenlüfterrad ausgeführt sein. Das Lüfterrad kann propellerartig oder als ein Propeller ausgebildet sein. Das Lüfterrad kann auch als ein Impeller ausgeführt sein und dann vorzugsweise von wenigstens einem Impellergehäuse ummantelt sein. Das Gehäuse weist dabei insbesondere wenigstens eine Ansaugöffnung und wenigstens eine Auslassöffnung auf. Das Lüfterrad ist insbesondere dazu geeignet und ausgebildet, von axial anzusaugen und nach radial auszublasen bzw. zu verdichten. Es können auch andere Anordnungen vorgesehen sein, beispielsweise kann von axial angesaugt und nach axial ausgeblasen werden oder auch von radial angesaugt und nach radial ausgeblasen werden. Das Lüfterrad kann als Radiallüfterrad oder Axiallüfterrad ausgeführt sein.

Die Lüftereinrichtung umfasst insbesondere wenigstens eine Antriebseinrichtung und beispielsweise wenigstens einen Elektromotor. Die Lüftereinrichtung ist insbesondere mit wenigstens einer Steuereinrichtung wirkverbunden und über diese ansteuerbar. Beispielsweise sind gezielt bestimmte Drehzahlen für das Lüfterrad einstellbar. Die Lüftereinrichtung umfasst insbesondere wenigstens einen Ansaugbereich und wenigstens einen Abgabebereich. Es ist möglich, dass der Ansaugbereich und/oder der Abgabebereich durch wenigstens eine Scheibe und/oder wenigstens ein Gehäuse oder dergleichen bereitgestellt werden.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine rein schematische Darstellung eines erfindungsgemäßen Atemtherapiegeräts in einer perspektivischen Ansicht;
- Fig. 2: eine rein schematische Darstellung eines üblichen Lüfterrades in einer perspektivischen Ansicht;
- Fug. 3: das Lüfterrad der Fig. 2 in einer Seitenansicht;
- Fig. 4: das Lüfterrad der Fig. 2 in einer Draufsicht;
- Fig. 5: ein erfindungsgemäßes Lüfterrad in einer perspektivischen Ansicht;
- Fug. 6: das Lüfterrad der Fig. 5 in einer Seitenansicht;
- Fig. 7: das Lüfterrad der Fig. 5 in einer Draufsicht;
- Fig. 8: ein stark schematisiertes Schaubild mit Kennlinien für Lüfterräder gemäß Fig. 2 bzw. Fig. 4
- Fig. 9: ein weiteres erfindungsgemäßes Lüfterrad in einer perspektivischen Ansicht;
- Fug. 10: das Lüfterrad der Fig. 9 in einer Seitenansicht;
- Fig. 11: das Lüfterrad der Fig. 9 in einer Draufsicht;
- Fig. 12: ein stark schematisiertes Schaubild mit Kennlinien für Lüfterräder gemäß Fig. 2 bzw. Fig. 9
- Fig. 13: ein weiteres erfindungsgemäßes Lüfterrad in einer perspektivischen Ansicht;
- Fig. 14: das Lüfterrad der Fig. 13 in einer Seitenansicht;
- Fig. 15: das Lüfterrad der Fig. 13 in einer Draufsicht;
- Fig. 16: ein stark schematisiertes Schaubild mit Kennlinien für Lüfterräder gemäß Fig. 2 bzw. Fig. 13
- Fig. 17: ein Lüfterrad analog zu dem der Fig. 5 jedoch mit gekrümmten Schaufeln

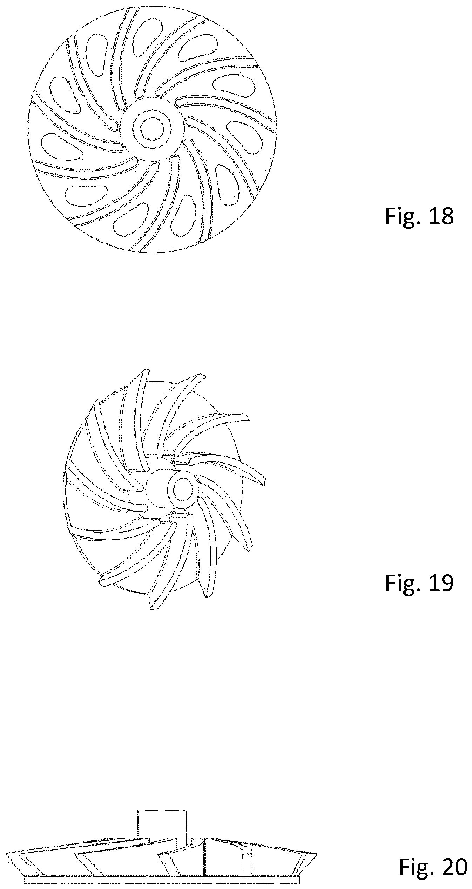

- Fig. 18: ein Lüfterrad analog zu dem der Fig. 7 jedoch mit gekrümmten Schaufeln
- Fig. 19: ein Lüfterrad analog zu dem der Fig. 9 jedoch mit gekrümmten Schaufeln
- Fig. 20: ein Lüfterrad analog zu dem der Fig. 10 jedoch mit gekrümmten Schaufeln

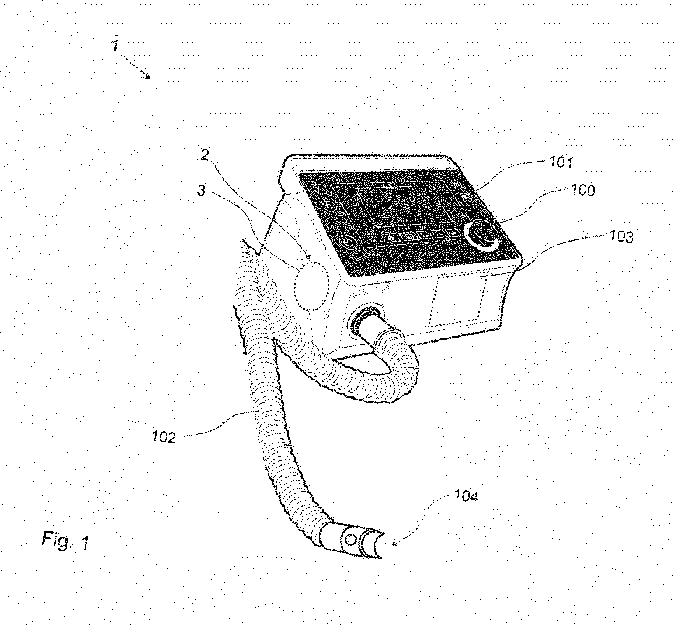

Die Figur 1 zeigt ein erfindungsgemäßes Atemtherapiegerät 1, welches beispielsweise ein Beatmungsgerät für klinische oder Heimanwendungen oder ein Notfallbeatmungsgerät oder ein Atemtherapiegerät oder ein Hustentherapiegerät ist. Das Gerät 1 ist mit einer hier nicht sichtbar im Geräteinneren untergebrachten Lüftereinrichtung 2 ausgestattet, mit der die Atemluftströmung für die Atemtherapie erzeugt wird. Die Lüftereinrichtung 2 ist mit einem erfindungsgemäßen Lüfterrad 3 ausgestattet. Zum Drehen des Lüfterrades 3 weist die Lüftereinrichtung 2 einen elektrischen Antrieb auf.

Die Lüftereinrichtung 2 wird über eine nicht sichtbar im Geräteinneren angeordnete Steuereinrichtung 103 angesteuert. Beispielsweise stellt die Steuereinrichtung 103 in Abhängigkeit der Therapievorgaben eine bestimmte Drehzahl des Lüfterrades 3 ein bzw. regelt die Lüfterdrehzahl auf einen Sollwert.

Das Atemtherapiegerät 1 ist hier mit einer Bedieneinrichtung 100 und einer Anzeigeeinrichtung 101 ausgestattet. Dabei erfolgt hier ein Teil der Bedienung über eine berührungsempfindliche Oberfläche der Anzeigeeinrichtung 101. Das Atemtherapiegerät 1 weist eine Schnittstelle zum Koppeln eines Schlauches 102 zur Beatmung bzw. Hustenunterstützung auf. Die mittels der Lüftereinrichtung 2 erzeugte Atemluftströmung wird über die Schlaucheinrichtung 102 dem Patienten zugeführt. An den Schlauch 102 kann eine hier nicht näher dargestellte Patientenschnittstelle und beispielsweise eine Atemmaske angeschlossen werden.

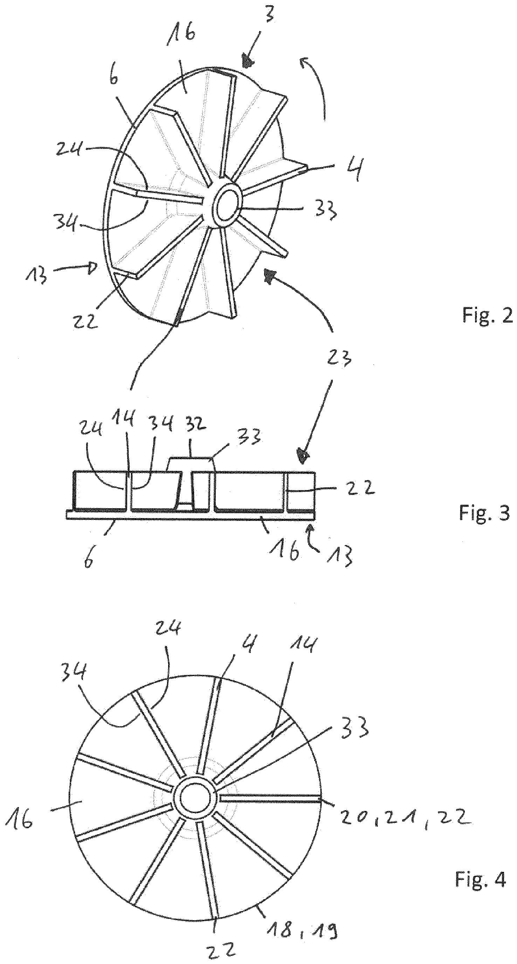

Die Figuren 2 bis 4 zeigen ein übliches Lüfterrad 3 in verschiedenen Ansichten. Das Lüfterrad 3 ist hier mit einer Mehrzahl von Schaufelelementen 4 und einer als Tragscheibe 16 ausgebildeten Scheibe 6 sowie einer Nabe 33 zur Verbindung mit einer Antriebswelle ausgestattet. Die Schaufelelemente 4 sind hier an der Tragscheibe 16 und vorzugsweise auch an der Nabe 33 befestigt und beispielsweise einstückig mit diesen verbunden. Das Lüfterrad 3 kann auch mit Tragscheibe 16 und Deckscheibe ausgeführt sein. Die Schaufelelemente 4 befinden sich dann zwischen Tragscheibe 16 und Deckscheibe.

Die bevorzugte Drehrichtung des Lüfterrades 3 ist hier durch einen Pfeil veranschaulicht. Die Schaufelelemente 4 weisen eine Saugseite 24 und eine Druckseite 34 auf. Für die hier gezeigte Drehrichtung des Lüfterrades 3 ergibt sich die hier gezeigte Ausrichtung von Saugseite 24 und Druckseite 34.

Das hier gezeigte Lüfterrad 3 ist beispielsweise so in die Lüftereinrichtung 2 integriert, dass von axial angesaugt und nach radial ausgeblasen wird. Dabei liegt die Ansaugseite an der axialen Seite 23.

In Fig. 3 und Fig. 4 ist erkennbar, dass die Materialstärke der Scheibe 6, recht dick ist und die Scheibe durchgehend ausgebildet ist. Ebenso sind die Schaufelelemente 4 materialmäßig recht dick bzw. in gleichmäßiger Stärke ausgebildet.

In der hier gezeigten Ausgestaltung ist das Lüfterrad 3 nur an einer axialen Seite 13 mit der Scheibe 6 ausgestattet. Dabei ist die als Tragscheibe 16 ausgebildete Scheibe 6 an derjenigen axialen Seite 13 des Lüfterrades 3 angeordnet, welche der Aufnahme der Welle des Motors dient.

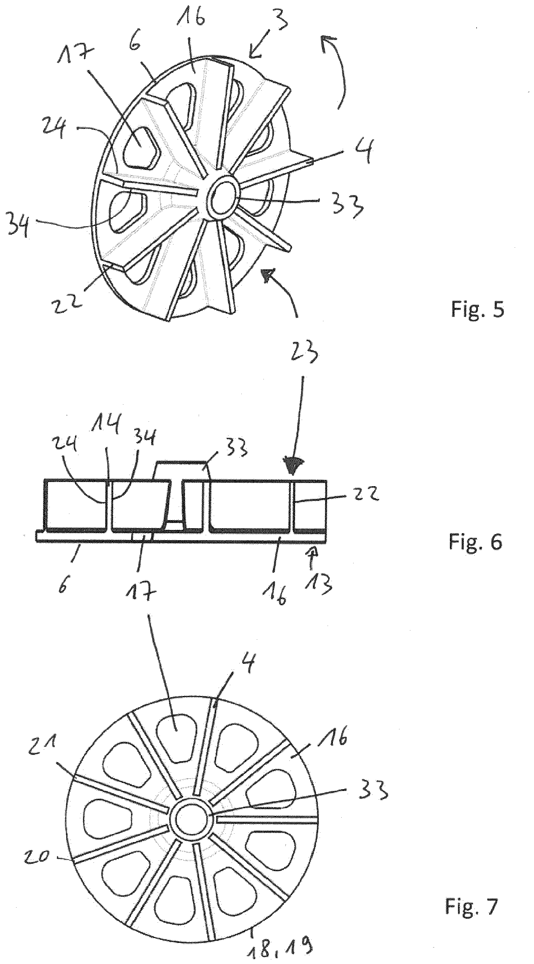

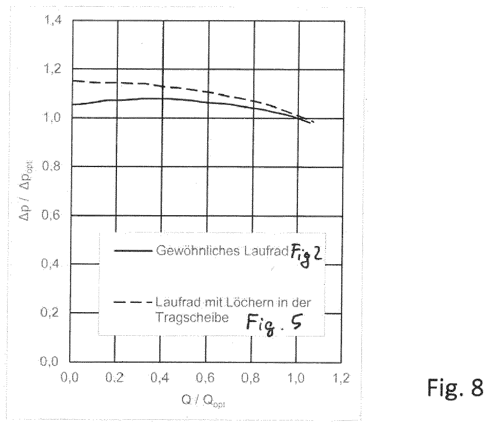

In den Figuren 5 bis 7 ist ein erfindungsgemäßes Lüfterrad 3 gezeigt, welches im Vergleich zu dem zuvor beschriebenen Lüfterrad 3, eine Materialaussparung 17 aufweist. Die Materialaussparung 17 kann als Dünnstelle oder Loch ausgebildet sein. Das Lüfterrad (3) weist eine Nabe (33) auf, die sich im Mittelpunkt 32 des Lüfterrades befindet, mit einer Tragscheibe 16, die sich ausgehend von der Nabe in radialer Richtung erstreckt und wobei die Tragscheibe einen Außenradius 19 aufweist, der einen Umfang 18 definiert, mit einer Mehrzahl von Schaufelelementen (4) die mit ihrer Basis (15) auf der Tragscheibe 16 angeordnet sind und sich ausgehend von der Nabe in radialer Richtung zu ihrer Schaufelspitze (22) erstrecken und sich ausgehend von der Basis 15 an der Deckscheibe in axialer Richtung erstrecken und dort eine Längsseite (14) definieren, wobei die Schaufelelemente 4 an den Schaufelspitzen (22) zumindest einen Außenradius (21) aufweisen der den Außenumfang (20) der Schaufelelemente (4) definiert. Die Schaufelelemente 4 und/oder die Tragscheibe 16 weisen wenigstens eine Aussparung 17 auf. Die Aussparung 17 ist in der Tragscheibe 16 beispielsweise als Loch oder Durchgangsloch und zwischen zwei Schaufelelementen 4 ausgebildet. Vorteil der Aussparungen 17 ist ein stark reduziertes Massenträgheitsmoment, d.h. es lässt sich besser Beschleunigen und Bremsen und bringt somit eine bessere Dynamik. In der Figur 8 sind zwei verschiedene Druckverläufe skizziert, bei denen der Druck gegen den Volumenstrom aufgetragen wurde. Die Achsen sind normiert auf optimalen Betriebspunkt der Konfiguration eines Lüfterrades aus dem SdT ohne Aussparungen. Der durchgezogene Druckverlauf entspricht dabei einem Lüfterrad 3 gemäß Fig. 2 -4 ohne Aussparungen 17 (SdT). Der grob gestrichelte Druckverlauf wurde bei einem Lüfterrad 3 gemäß Fig. 5 - 7 mit Aussparungen 17 erfasst. Die Kennlinie zeigt für das erfindungsgemäße Lüfterrad sogar mehr Druckaufbau. Entsprechend kann es bei kleinerer Drehzahl gefahren werden um den gleichen Betriebspunkt zu erreichen wie ein gewöhnliches Laufrad. Daraus würde eine zusätzliche Schallreduktion hervorgehen.

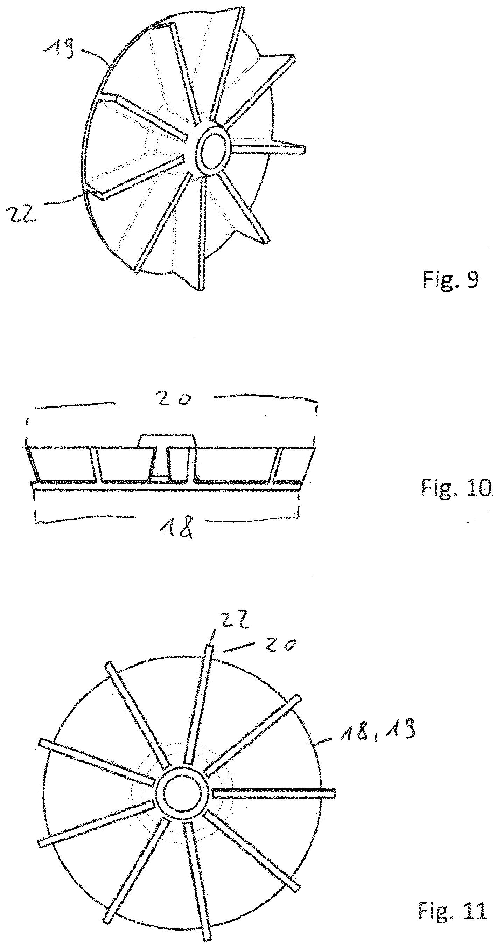

In den Figuren 9 bis 11 ist ein erfindungsgemäßes Lüfterrad 3 gezeigt, welches im Vergleich zu dem Lüfterrad 3 gemäß Fig. 2, eine Materialaussparung 17 aufweist. Die Aussparung 17 ist in dem Material des Schaufelelementes 4 und an dem Umfang der Tragscheibe ausgebildet. Die Aussparung 17 befindet sich an Teilen der Schaufelspitze 22 und an dem Umfang der Tragscheibe und daher ist der Außenradius (21) größer als der Außenradius 19 der Tragscheibe.

Entsprechend ist der Umfang 18 der Tragscheibe kleiner als der Umfang 20 der Schaufelelemente.

Die Aussparung 17 ist an der Schaufelspitze 22 derart ausgebildet und angeordnet, dass die Schaufelspitze 22 in axialer Richtung abgeschrägt ist. Dabei ist der Außenradius 21 an der Basis 15 kleiner als an der Längsseite 14. Der Außenradius 21 an der Basis 15 kann dem Außenradius 19 der Tragscheibe entsprechen.

Die axiale Abschrägung liegt in einem Bereich zwischen 1° und 50°. Die axiale Abschrägung liegt bevorzugt in einem Bereich zwischen 1° und 25° oder 2° und 20°.Die axiale Abschrägung liegt bevorzugt auch in einem Bereich zwischen 3° und 16° oder 4° und 7°. Gemäß den Fig. 9 - 11 ändert sich der Durchmesser über die axiale Länge des Laufrades und die Tragscheibe ist kleiner im Durchmesser als der der Schaufelelemente.

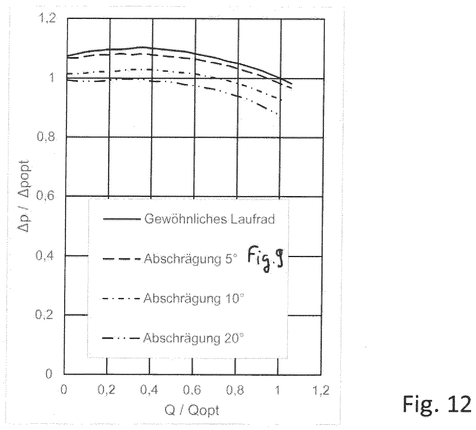

In der Figur 12 sind vier verschiedene Druckverläufe skizziert, bei denen der Druck gegen den Volumenstrom aufgetragen wurde. Die Achsen sind normiert auf optimalen Betriebspunkt der Konfiguration eines Lüfterrades aus dem SdT ohne Aussparungen. Der durchgezogene Druckverlauf entspricht dabei einem Lüfterrad 3 gemäß Fig. 2 -4 ohne Aussparungen 17 (SdT). Der grob gestrichelte Druckverlauf wurde bei einem Lüfterrad 3 gemäß Fig. 9 - 11 mit einer Abschrägung von 5° erfasst. Im Vergleich zu dem Laufrad ohne Abschrägung gemäß Fig. 2 zeigt sich nur ein geringfügiger Kennlinienunterschied bezogen auf den Druck. Das bedeutet, dass bei einem deutlich reduzierten Massenträgheitsmoment die Kennlinie praktisch gleichbleibt. Die Leistungsaufnahme für abgeschrägtes Laufrad ist sogar geringer, sodass der Wirkungsgrad gleichbleibt. Die darunterliegenden Kennlinien entsprechen einer Abschrägung von 10° bzw. 20°. Bei einer Abschrägung von 10 -15° ergibt sich eine geringere Kennlinie als für Laufrad ohne Abschrägung gemäß Fig.2. Bei einer Abschrägung von 20° ergibt sich eine deutlich geringere Kennlinie als für Laufrad ohne Abschrägung gemäß Fig.2, wobei die Werte noch immer akzeptabel sind. Die Verluste des Drucks in der Kennlinie sind allesamt akzeptabel, wenn man die Einsparung des Massenträgheitsmoments berücksichtigt.

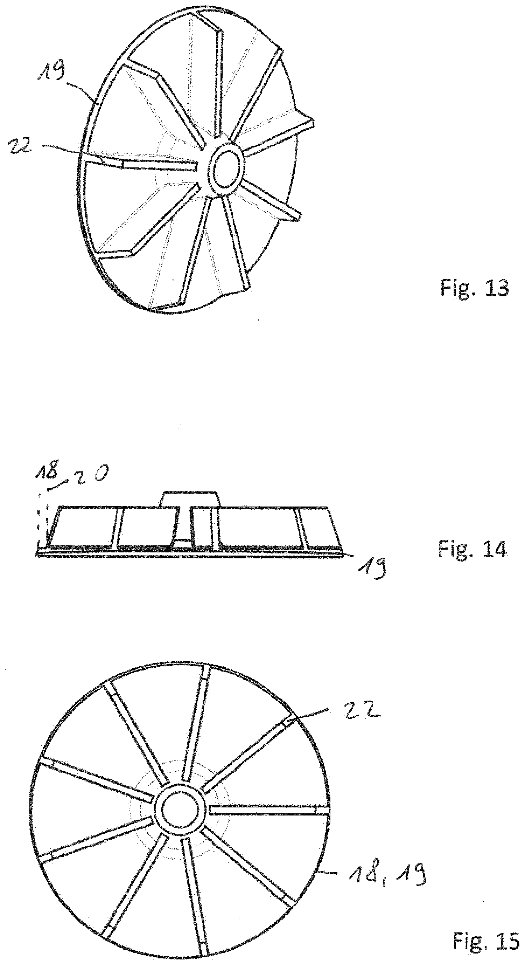

In den Figuren 13 bis 15 ist ein erfindungsgemäßes Lüfterrad 3 gezeigt, welches im Vergleich zu dem Lüfterrad 3 gemäß Fig. 2, eine Materialaussparung 17 aufweist. Die Aussparung 17 ist in dem Material des Schaufelelementes 4 ausgebildet. Die Aussparung 17 befindet sich an der Schaufelspitze 22 und daher ist der Außenradius (21) geringer als der Außenradius 19 der Tragscheibe. Entsprechend ist der Umfang 18 der Tragscheibe größer als der Umfang 20 der Schaufelelemente. Der Durchmesser ändert sich über die axiale Länge des Laufrades. Dabei ist der Durchmesser der Tragscheibe größer als der Schaufeldurchmesser.

Die Aussparung 17 ist an der Schaufelspitze 22 derart ausgebildet und angeordnet, dass die Schaufelspitze 22 in axialer Richtung abgeschrägt ist. Dabei ist der Außenradius an der Basis größer als an der Längsseite 14. Die axiale Abschrägung liegt in einem Bereich zwischen 1° und 50°. Die axiale Abschrägung liegt bevorzugt in einem Bereich zwischen 1° und 25° oder 2° und 20°.

Die axiale Abschrägung liegt bevorzugt auch in einem Bereich zwischen 3° und 16° oder 4° und 7°.

Ein Vorteil ist ein reduziertes Massenträgheitsmoment, d.h. das erfindungsgemäße Laufrad ist besser zu Beschleunigen und zu Bremsen. Ein weiterer Vorteil ist, dass sich der Schwerpunkt des Laufrades in Richtung der Tragscheibe (und damit zum Motor) verschiebt, dies ist besser für die Lagerung.

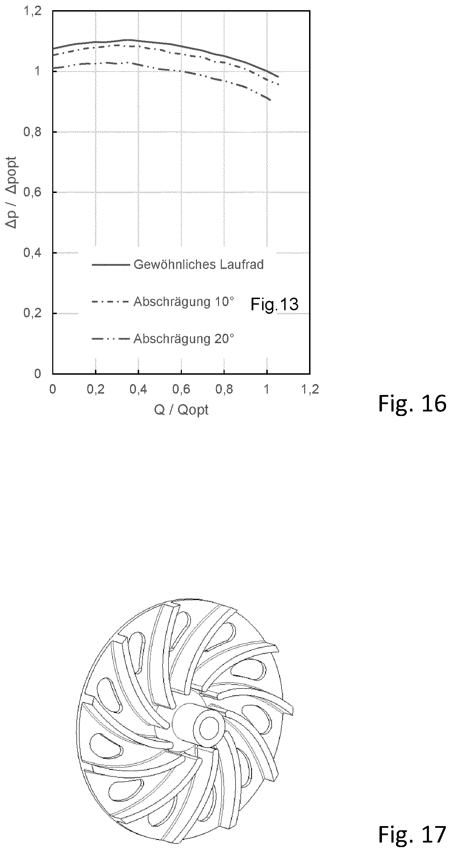

In der Figur 16 sind drei verschiedene Druckverläufe skizziert, bei denen der Druck gegen den Volumenstrom aufgetragen wurde. Die Achsen sind normiert auf optimalen Betriebspunkt der Konfiguration eines Lüfterrades aus dem SdT ohne Aussparungen. Der durchgezogene Druckverlauf entspricht dabei einem Lüfterrad 3 gemäß Fig. 2 - 4 ohne Aussparungen 17 (SdT). Der darunterliegende Druckverlauf wurde bei einem Lüfterrad 3 gemäß Fig. 13 -15 mit einer Abschrägung von 10° erfasst. Im Vergleich zu dem Laufrad ohne Abschrägung gemäß Fig. 2 zeigt sich nur ein geringfügiger Kennlinienunterschied bezogen auf den Druck. Das bedeutet, dass bei einem deutlich reduzierten Massenträgheitsmoment die Kennlinie praktisch gleichbleibt. Die Leistungsaufnahme für abgeschrägtes Laufrad ist sogar geringer, sodass der Wirkungsgrad gleichbleibt. Die darunterliegende Kennlinie entsprechen einer Abschrägung von 20° Bei einer Abschrägung von 20° ergibt sich eine deutlich geringere Kennlinie als für Laufrad ohne Abschrägung gemäß Fig.2, wobei die Werte noch immer akzeptabel sind. Die Verluste des Drucks in der Kennlinie sind allesamt akzeptabel, wenn man die Einsparung des Massenträgheitsmoments berücksichtigt. Für eine Abschrägung von 5° zeigte sich sogar eine (nicht dargestellte) Kennlinie, die der Kennlinie aus dem SdT entspricht. Der Wirkungsgrad wäre hier sogar erhöht.

Für alle Ausführungsformen gilt, dass auch eine zusätzliche Scheibe 6 als Deckscheibe 26 verwendet werden kann. Diese bedeckt dann die Schaufelelemente 4.

Die Schaufelelemente 4 sind hier gerade als 90°-Schaufeln ausgeführt; jedoch sind gebogene Schaufeln ebenso denkbar.

Die Schaufelelemente 4 können Winglets aufweisen.

Die Aussparungen können kombiniert an den Schaufelelementen und den Scheiben 16 bzw. 26 verwendet werden.

### Bezugszeichenliste:

- 1: Atemtherapiegerät
- 2: Lüftereinrichtung
- 3: Lüfterrad
- 4: Schaufelelement
- 5: Winglet
- 6: Scheibe
- 13: Seite
- 14: Längsseite
- 15: Basis
- 16: Tragscheibe
- 17: Aussparung / Loch
- 18: Umfang Tragscheibe
- 19: Außenradius Tragscheibe
- 20: Umfang Schaufelelement
- 21: Außenradius Schaufelelement
- 22: Schaufelspitze
- 23: Seite
- 24: Saugseite
- 26: Deckscheibe
- 32: Rotationsachse Lüfterrad
- 33: Nabe
- 34: Druckseite
- 100: Bedieneinrichtung
- 101: Anzeigeeinrichtung
- 102: Schlauch
- 103: Steuereinrichtung
- 104: Patientenschnittstelle
- 200: Druck
- 201: Volumenstrom
- 202: Wirkungsgrad

## Patentansprüche

1. Atemtherapiegerät (1) mit wenigstens einer Lüftereinrichtung (2) zum Erzeugen einer Atemluftströmung für die Durchführung einer Atemtherapie, wobei die Lüftereinrichtung (2) wenigstens ein drehbares Lüfterrad (3) mit einer zentralen Nabe (33) aufweist, mit einer Tragscheibe (16), die sich ausgehend von der Nabe im Wesentlichen in radialer Richtung erstreckt und wobei die Tragscheibe einen Außenradius (19) aufweist, der einen Umfang (18) definiert, mit einer Mehrzahl von Schaufelelementen (4) die mit ihrer Basis (15) auf der Tragscheibe (16) angeordnet sind und sich ausgehend von der Nabe im Wesentlichen in radialer Richtung zu ihrer Schaufelspitze (22) erstrecken und sich ausgehend von der Basis (15) an der Tragscheibe in axialer Richtung erstrecken und dort eine Längsseite (14) definieren, wobei die Schaufelelemente (4) an den Schaufelspitzen (22) zumindest einen Außenradius (21) aufweisen **dadurch gekennzeichnet, dass** die Schaufelelemente (4) und/oder die Tragscheibe (16) wenigstens eine Aussparung (17) aufweisen.

2. Atemtherapiegerät (1) nach Anspruch 1, wobei sich die Schaufelelemente (4) ausgehend von der Nabe zu ihrer Schaufelspitze (22) in gerader oder in gekrümmter Weise erstrecken.

3. Atemtherapiegerät (1) nach zumindest einem der vorhergehenden Ansprüche, wobei die Aussparung (17) in dem Material des Lüfterrades (3) ausgebildet ist.

4. Atemtherapiegerät (1) nach zumindest einem der vorhergehenden Ansprüche, wobei mehrere Aussparungen (17) in Umfangsrichtung des Lüfterrades (3) nebeneinander ausgebildet sind.

5. Atemtherapiegerät (1) nach zumindest einem der vorhergehenden Ansprüche, wobei mehrere Aussparungen (17) in Radialrichtung des Lüfterrades (3) hintereinander ausgebildet sind.

6. Atemtherapiegerät (1) nach zumindest einem der vorhergehenden Ansprüche, wobei die Aussparung (17) in der Tragscheibe (16) ausgebildet ist.

7. Atemtherapiegerät (1) nach zumindest einem der vorhergehenden Ansprüche, wobei zumindest ein Durchgangsloch (17) in der Tragscheibe (16) zwischen zwei Schaufelelementen (4) ausgebildet ist.

8. Atemtherapiegerät (1) nach zumindest einem der beiden vorhergehenden Ansprüche, wobei die Schaufelelemente (4) innerhalb eines Umfangs (18) der Scheibe (6) angeordnet sind und/oder wobei die Schaufelelemente (4) nicht über den Umfang der Scheibe (6) hinausstehen.

9. Atemtherapiegerät (1) nach zumindest einem der vorhergehenden Ansprüche, wobei die Aussparung (17) in dem Material des Schaufelelementes (4) ausgebildet ist.

10. Atemtherapiegerät (1) nach zumindest einem der vorhergehenden Ansprüche, wobei die Aussparung (17) an der Schaufelspitze (22) ausgebildet ist und daher der Außenradius (21) geringer ist als der Außenradius (19) der Tragscheibe.

11. Atemtherapiegerät (1) nach zumindest einem der vorhergehenden Ansprüche, wobei der Umfang (18) der Tragscheibe größer ist als der Umfang (20) der Schaufelelemente.

12. Atemtherapiegerät (1) nach zumindest einem der vorhergehenden Ansprüche, wobei die Aussparung (17) in dem Bereich des Außenradius (19) der Tragscheibe ausgebildet ist und daher der Außenradius (19) geringer ist als der Außenradius (21) der Schaufelelemente.

13. Atemtherapiegerät (1) nach zumindest einem der vorhergehenden Ansprüche, wobei der Umfang (20) der Schaufelelemente größer ist als der Umfang (18) der Tragscheibe.

14. Atemtherapiegerät (1) nach zumindest einem der vorhergehenden Ansprüche, wobei die Aussparung (17) an der Schaufelspitze (22) derart ausgebildet und angeordnet ist, dass die Schaufelspitze (22) in axialer Richtung abgeschrägt ist.

15. Atemtherapiegerät (1) nach zumindest einem der vorhergehenden Ansprüche, wobei die axiale Abschrägung in einem Bereich zwischen 1° und 50° oder in einem Bereich zwischen 1° und 25° liegt.

16. Atemtherapiegerät (1) nach zumindest einem der vorhergehenden Ansprüche, wobei die Aussparung (17) an der Schaufelspitze (22) derart ausgebildet und angeordnet ist, dass die Schaufelspitze (22) in axialer Richtung gekrümmt oder gebogen ist.

17. Atemtherapiegerät (1) nach zumindest einem der vorhergehenden Ansprüche, wobei die Aussparung (17) an der Schaufelspitze (22) derart ausgebildet und angeordnet ist, dass das Schaufelelement an der Schaufelspitze (22) zumindest zwei unterschiedliche Außenradien (21) aufweist.

18. Atemtherapiegerät (1) nach zumindest einem der vorhergehenden Ansprüche, wobei ein geringerer Außenradius (21) des Schaufelelementes an der Schaufelspitze (22) an der oder nahe der Basis (15) oder nahe der Längsseite (14) des Schaufelelementes (4) angeordnet ist.

19. Atemtherapiegerät (1) nach zumindest einem der vorhergehenden Ansprüche, wobei ein geringerer Außenradius (21) zwischen der Längsseite (14) und der Basis (15) des Schaufelelementes (4) angeordnet ist.

20. Atemtherapiegerät (1) nach zumindest einem der vorhergehenden Ansprüche, wobei die Aussparungen eine geringere Materialstärke aufweisen als die Scheibe (6) oder die Schaufelelemente (4).

21. Lüfterrad (3) für ein Atemtherapiegerät (1) nach zumindest einem der vorhergehenden Ansprüche.
